# EUROPEAN PATENT SPECIFICATION

(11) **EP 1 737 373 B1**
(45) Date of publication and mention of the grant of the patent: **22.09.2010**
(21) Application number: 05718684.3
(22) Date of filing: 11.04.2005
(51) Int. Cl.: A61B 18/20

(54) **A DEVICE FOR THE TREATMENT OF SKIN BY MEANS OF A RADIATION BEAM**
VORRICHTUNG FÜR DIE HAUTBEHANDLUNG MITTELS BESTRAHLUNG
DISPOSITIF POUR LE TRAITEMENT DE LA PEAU PAR UN FAISCEAU DE RAYONNEMENT

(30) Priority: 15.04.2004 EP 04101548
(43) Date of publication of application: 03.01.2007
(73) Proprietor: Koninklijke Philips Electronics N.V., 5621 BA Eindhoven (NL)
(72) Inventor: VAN HAL, Robbert, A., M., NL-5656 AA Eindhoven (NL); HOUBOLT, Erik, NL-5656 AA Eindhoven (NL); ZUIDERVAART, Jasper, NL-5656 AA Eindhoven (NL); NUIJS, Antonius, M., NL-5656 AA Eindhoven (NL); NIEHAUS, Mathijs, NL-5656 AA Eindhoven (NL); ACKERMANS, Paul, A., J., NL-5656 AA Eindhoven (NL); VERHAGEN, Rieko, NL-5656 AA Eindhoven (NL)
(74) Representative: Wolfs, Marc Johannes Maria
(86) International application number: PCT/IB2005/051176
(87) International publication number: WO 2005/099607

(56) References cited:
- EP-A- 1 226 787
- WO-A-00/62700
- US-A- 4 733 660
- US-B1- 6 682 524

## Description

The invention relates to a device for the treatment of skin by means of a radiation beam, which device comprises a radiation source for generating the radiation beam, a beam exit window, an optical detecting system for determining a target position of the radiation beam relative to the skin, and a beam manipulator for positioning the radiation beam in the target position, the radiation beam and the detecting system having a first and a second optical axis, respectively.

A device for the treatment of skin of the kind mentioned in the opening paragraph is known from WO00/62700. The known device is a device for cutting or removing hairs by means of laser beam pulses. The laser beam is generated by means of a laser source in the device. The beam manipulator of the known device comprises two tiltable mirrors by means of which the laser beam can be deflected in two mutually perpendicular X-and Y-directions which each extend parallel to the beam exit window and the skin surface. The optical detecting system of the known device comprises an illumination member for illuminating a portion of the skin present before the beam exit window. Light reflected by the illuminated skin portion is detected by a CMOS image sensor of the optical detecting system. The known device further comprises a control unit which controls the laser source and the tilting position of the mirrors of the beam manipulator. The control unit comprises a processor which determines the target position of the laser beam relative to the skin, i.e. the position in which the laser beam should cut a detected hair, on the basis of an image of the skin detected by the image sensor. The control unit supplies a control signal to the beam manipulator which corresponds with a desired tilting position of the mirrors corresponding with the desired target position. After adjusting the desired tilting position of the mirrors, the control unit activates the laser source, so that the detected hair is cut in the desired target position.

In view of the relatively small diameters of the hairs to be cut, the target positions should be determined in an accurate manner, and the laser beam should be accurately positioned in the target positions by means of the beam manipulator. A disadvantage of the known device is that errors in the tilting position of the mirrors of the beam manipulator may occur as a result of, for example, temperature increases of the device, manufacturing tolerances or mechanical play. As a result of said errors the laser beam may be positioned by the beam manipulator with insufficient accuracy, so that the target position determined by the detecting system may be missed by the laser beam. As a result, the efficiency of the known device may be affected, and the occurrence of unwanted side effects such as skin irritations and injuries may increase.

Document WO 00/62700 discloses a device as described in the preamble of claim 1.

It is an object of the present invention to provide a device for the treatment of skin of the kind mentioned in the opening paragraph, in which the accuracy with which the laser beam is positioned in a target position determined by means of the detecting system is considerably increased, so that the above mentioned disadvantages of the know device are prevented.

In order to achieve this object, a device for the treatment of skin in accordance with the invention is **characterized in that**, seen from the radiation source towards the beam exit window, the first and the second optical axis coincide starting at a position between the radiation source and the beam manipulator. Since the first optical axis of the radiation beam and the second optical axis of the detecting system coincide starting at a position between the radiation source and the beam manipulator, the optical path along which the radiation beam is guided to the target position and the optical path along which the detecting system determines the target position are both manipulated in an identical way by the beam manipulator. As a result, if an error in the position of the optical components of the beam manipulator occurs, said error will lead to a first error in the position of the radiation beam relative to the skin set by the beam manipulator and to a substantially identical second error in the target position determined by the detecting system. Said substantially identical first and second errors will compensate each other, so that the error in the position of the optical components of the beam manipulator will substantially not lead to a deviation between the position of the laser beam relative to the skin set by the beam manipulator and the target position determined by the detecting system.

A particular embodiment of a device in accordance with the invention is **characterized in that** the device comprises a dichroic beam splitter in said position between the radiation source and the beam manipulator. The dichroic beam splitter is a practical optical component by means of which, seen in a direction from the beam manipulator towards the radiation source, a necessary split-up is made of the optical paths of the radiation beam and the detecting system, and by means of which, seen in a direction from the radiation source towards the beam manipulator, a necessary combination of said optical paths is made.

A particular embodiment of a device in accordance with the invention is **characterized in that** the optical detecting system comprises an image sensor, the second optical axis extending from the image sensor towards the beam exit window. In this embodiment, the image sensor is used to make an image of a portion of the skin which is present before the beam exit window. If the device is for example used as a device for shortening or cutting hairs, the position and orientation of the hairs present on said skin portion is determined from said image by means of a control unit of the device. From said position and orientation of the hairs, the control unit determines a number of successive target positions for the radiation beam and adjusts the beam manipulator into a number of successive positions corresponding with said target positions.

A particular embodiment of a device in accordance with the invention is **characterized in that** the optical detecting system comprises an auxiliary radiation source for generating an auxiliary radiation beam, and a detector for detecting a characteristic optical signal coming from a hair under the influence of the auxiliary radiation beam, the second optical axis extending from the auxiliary radiation source towards the beam exit window. In this embodiment, the device is a device for shortening or cutting hairs. Said detector of the detecting system is preferably arranged close to the beam exit window. Since the first optical axis of the radiation beam and the second optical axis of the detecting system coincide starting at a position between the radiation source and the beam manipulator, the auxiliary radiation beam will always hit the skin in a position where the radiation beam would hit the skin if the radiation source were switched on. If the auxiliary radiation beam hits a hair, the hair will for example irradiate or reflect said characteristic optical signal, which will immediately be detected by said detector of the detecting system. Upon detection of said characteristic optical signal, the radiation source will be activated to generate a radiation pulse, which will hit the hair in the position detected by means of the auxiliary radiation beam.

A further embodiment of a device in accordance with the invention is **characterized in that** the detector is a two-photon fluorescence detector for detecting a characteristic two-photon fluorescence signal irradiated by a hair under the influence of the auxiliary radiation beam. By means of said characteristic two-photon fluorescence signal a hair can be distinguished in a practical and reliable manner, so that the detecting system has a high reliability.

A particular embodiment of a device in accordance with the invention is **characterized in that** the device is a device for shortening hairs, wherein the radiation source is a laser source.

Embodiments of a device for the treatment of skin in accordance with the invention will be described in detail in the following with reference to the drawings, in which
Fig. 1 schematically shows a first embodiment of a device for the treatment of skin in accordance with the invention; and
Fig. 2 schematically shows a second embodiment of a device for the treatment of skin in accordance with the invention.

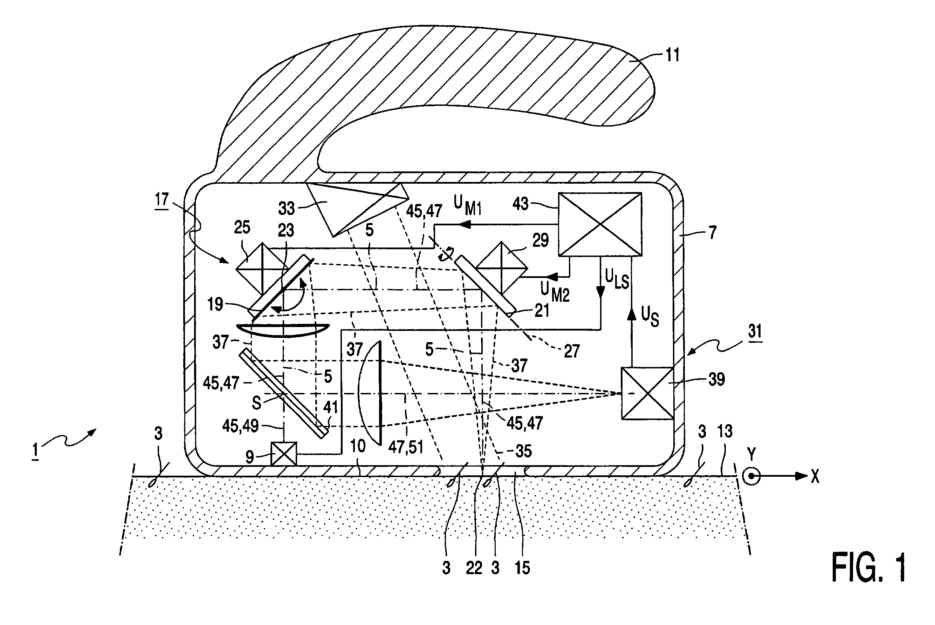

Figure 1 shows a first embodiment of a device 1 for the treatment of skin in accordance with the invention. The device 1 is a device for shortening or cutting hairs 3 growing from human skin 13 by means of a laser beam 5. The device 1 comprises a housing 7 which accommodates a laser source 9 for generating the laser beam 5. The housing 7 comprises a skin contact surface 10 and a grip 11. During operation the skin contact surface 10 lies against the skin 13. The laser beam 5 exits the housing 7 via a beam exit window 15 provided in the skin contact surface 10.

The housing 7 further accommodates a beam manipulator 17 comprising first and second inclined tiltable mirrors 19 and 21 for positioning the laser beam 5 in a target position 22 relative to the skin 13. The first mirror 19 is tiltable about a first tilting axis 23 by means of a first electrical actuator 25, and the second mirror 21 is tiltable about a second tilting axis 27 by means of a second electrical actuator 29. The first tilting axis 23 extends in the mirror surface of the first mirror 19 and parallel to a Y-direction, which extends parallel to the skin contact surface 10. The second tilting axis 27 extends in the mirror surface of the second mirror 21 and perpendicularly to the Y-direction. The laser beam 5 generated by the laser source 9 is guided and reflected towards the beam exit window 15 via the first and the second mirror 19, 21. A tilting movement of the first mirror 19 leads to a displacement of the target position 22 of the laser beam 5 over the skin 13 parallel to an X-direction, which extends parallel to the skin contact surface 10 and perpendicularly to the Y-direction. A tilting movement of the second mirror 21 leads to a displacement of the target position 22 of the laser beam 5 over the skin 13 parallel to the Y-direction.

The housing 7 further accommodates an optical detecting system 31 for determining the target position 22 of the laser beam 5 relative to the skin 13. The detecting system 31 comprises a light source 33 which generates a light beam 35 in the visual part of the spectrum for illuminating a portion of the skin 13 which is present in front of the beam exit window 15. A light beam 37 reflected by the illuminated skin 13 is guided and reflected towards a CMOS image sensor 39 of the detecting system 31 via the first and the second mirror 19, 21 and via a dichroic beam splitter 41 which is provided in a position between the laser source 9 and the first mirror 19 of the beam manipulator 17. The dichroic beam splitter 41 is transparent for the laser beam 5, which has a wavelength in the near infra red spectrum region, and the dichroic beam splitter 41 is reflective for the visual part of the spectrum. It is noted that, instead of a dichroic beam splitter, another type of beam splitter can be used such as a polarizing beam splitter.

The CMOS image sensor 39 generates an output signal uₛ which corresponds to an image of the portion of the skin 13 which is present in front of the beam exit window 15. The output signal uₛ is supplied to an electrical control unit 43 of the device 1, which controls the tilting positions of the first and the second mirror 19, 21 and the operation of the laser source 9. From the signal uₛ the control unit 43 determines the position and orientation of the hairs 3, which are present on the portion of the skin 13 in front of the beam exit window 15, on the basis of an image recognition method. On the basis of said position and orientation of the hairs 3, the control unit 43 determines a number of successive target positions 22 of the laser beam 5 on the hairs 3, i.e. a number of successive positions on the hairs 3 wherein the laser source 9 should generate a laser pulse in order to cut the hairs 3. Further details about the control unit 43 and the image recognition method will not be described here. Reference is made to WO-A-00/62700 which describes embodiments of a control unit and an image recognition method which might also be used in the device 1. The control unit 43 generates a first output signal u_{M1}, which is supplied to the first actuator 25 and corresponds to an X-position of the desired target position 22, a second output signal u_{M2}, which is supplied to the second actuator 29 and corresponds to a Y-position of the desired target position 22, and a third output signal u_{LS}, which is supplied to the laser source 9 in order to activate the laser source 9. For each desired target position 22 determined on the basis of the signal u_{S}, the control unit 43 adjusts the first and the second mirror 19, 21 into positions corresponding with said desired target position 22 and, after adjustment of the mirrors 19, 21, activates the laser source 9 in order to generate a laser pulse having a pulse duration and energy density sufficient to cut the hair 3 in said target position 22.

The laser beam 5 has a first optical axis 45 which extends from the laser source 9 via the beam splitter 41 and via the first and the second mirror 19, 21 of the beam manipulator 17 towards the beam exit window 15. The optical detecting system 31 has a second optical axis 47 which extends from the image sensor 39 via the beam splitter 41 and via the first and the second mirror 19, 21 of the beam manipulator 17 towards the beam exit window 15. According to the invention the optical components of the device 1 are arranged in such mutual positions that, seen from the laser source 9 towards the beam exit window 15, the first optical axis 45 and the second optical axis 47 coincide starting at a position between the laser source 9 and the beam manipulator 17. In the first embodiment of the device 1 the invention is realized in that the portion 49 of the first optical axis 45, extending between the laser source 9 and the dichroic beam splitter 41, and the portion 51 of the second optical axis 47, extending between the image sensor 39 and the dichroic beam splitter 41, are perpendicular relative to each other, and in that the beam splitter 41 extends through a point of intersection S of said two portions 49, 51 and is arranged at an angle of 45° relative to each of said portions 49 and 51. As a result, seen towards the beam exit window 15, the first and the second optical axis 45 and 47 coincide starting at said point of intersection S, i.e. the first and the second optical axis 45, 47 coincide between the point of intersection S and the first mirror 19, between the first mirror 19 and the second mirror 21, and further from the second mirror 21 towards and beyond the beam exit window 15. Since the first and the second optical axis 45, 47 coincide from said point of intersection S which, seen towards the beam exit window 15, is present before the beam manipulator 17, the laser beam 5 and the light beam 37 detected by the image sensor 39 are manipulated identically by the beam manipulator 17. If an error occurs in the tilting positions of the mirrors 19, 21 as a result of, for example, temperature increases in the device 1, manufacturing tolerances or mechanical play, i.e. if certain predetermined values of the signal u_{M1}, and u_{M2} do not exactly lead to the required predetermined adjustments of the mirrors 19, 21, said error will lead to a first error in the target position 22 of the laser beam 5 actually set by the beam manipulator 17 and will lead to a second error in the target position determined by the control unit 43 on the basis of the image detected by the detecting system 31. Since the laser beam 5 and the light beam 37 are manipulated identically by the beam manipulator 17, said first and said second error are substantially identical and will compensate each other. As a result, the error in the tilting positions of the mirrors 19, 21 will substantially not lead to a deviation between the target position determined by the control unit 43 and the detecting system 31 and the target position actually set by the beam manipulator 17. Accordingly, the target positions are set in an accurate manner by means of the beam manipulator 17, so that the risk that the target position on a hair 3 is missed by the laser beam 5 is considerably reduced.

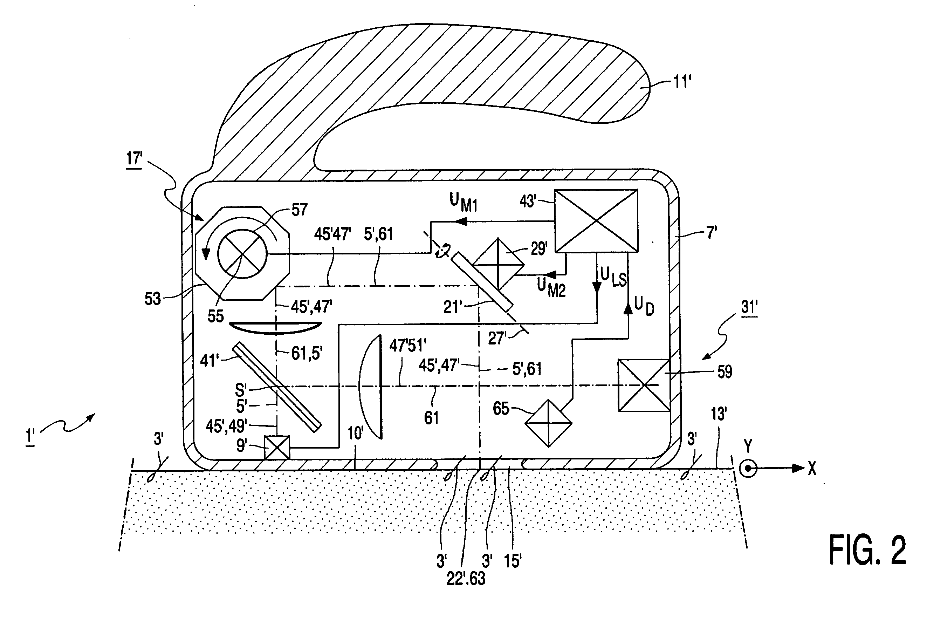

Figure 2 shows a second embodiment of a device 1' for the treatment of skin in accordance with the invention. Like the device 1 described herebefore, the device 1' is a device for shortening or cutting hairs 3' growing from human skin 13' by means of a laser beam 5'. In fig. 2 parts of the device 1' corresponding with parts of the device 1 described herebefore are indicated with corresponding reference numbers. These corresponding parts will not be discussed further. In the following, only the main differences between the device 1' and the device 1 will be discussed.

The device 1' comprises a beam manipulator 17' having a first mirror 53 and a second mirror 21' which is substantially identical to the second mirror 21 of the device 1. The first mirror 53 is a polygonal mirror which is rotatable about an axis of rotation 55 extending parallel to the Y-direction by means of an electrical motor 57. The laser beam 5' generated by the laser source 9' is guided and reflected towards the beam exit window 15' via the first and the second mirror 53 and 21'. The rotational movement of the polygonal mirror 53 leads to a scanning movement of the target position 22' of the laser beam 5' over the skin 13' via a scanning path parallel to the X-direction, while the tilting movement of the second mirror 21' leads to a displacement of said scanning path parallel to the Y-direction.

The device 1' further comprises an optical detecting system 31' for determining the target positions 22' of the laser beam 5' in which the laser source 9' should be switched on. The detecting system 31' comprises an auxiliary laser source 59 for generating an auxiliary laser beam 61. The auxiliary laser beam 61 is guided and reflected towards the beam exit window 15' via the dichroic beam splitter 41' and via the first and the second mirror 53, 21'. The dichroic beam splitter 41' is transparent for the laser beam 5', which has a wavelength in the near infra red spectrum region, but is reflective for the wavelength of the auxiliary laser beam 61. The detecting system 31' has a second optical axis 47' which extends from the auxiliary laser source 59 via the beam splitter 41' and via the first and the second mirror 53 and 21' towards the beam exit window 15'. Like in the device 1, the optical components of the device 1' are arranged in such mutual positions that, seen from the laser source 9' towards the beam exit window 15', the first optical axis 45' of the laser beam 5' and the second optical axis 47' of the detecting system 31' coincide starting at a position between the laser source 9' and the beam manipulator 17', i.e. in the embodiment shown starting at the point of intersection S' of the mutually perpendicular portions 49' and 51' of the first and second optical axes 45' and 47'. As a result, the laser beam 5' and the auxiliary laser beam 61 are manipulated identically by means of the beam manipulator 17', so that the target position 22' of the laser beam 5' relative to the skin 13' and the target position 63 of the auxiliary laser beam 61 relative to the skin 13' always coincide.

The detecting system 31' further comprises a detector 65 for detecting a characteristic two-photon fluorescence signal irradiated by a hair under the influence of the auxiliary laser beam 61. The detector 65 is arranged in a position close to the beam exit window 15'.

The device 1' further comprises a control unit 43' which controls the rotational speed of the first mirror 53, the tilting position of the second mirror 21', and the operation of the laser source 9'. During operation, the control unit 43' generates a first output signal u_{M1}, which is supplied to the motor 57 of the first mirror 53 and corresponds to a desired scanning speed of the target positions 22', 63 over the skin 13' parallel to the X-direction. The control unit 43' further generates a second output signal u_{M2}, which is supplied to the electrical actuator 29' of the second mirror 21' and corresponds to a desired Y-position of the scanning path of the target positions 22', 63. After each complete scanning movement of the target positions 22', 63 parallel to the X-direction, which corresponds with a rotation of the first mirror 53 over an angle of 60° since the first mirror 53 has 6 facets, the scanning path is adjusted over a relatively small distance parallel to the Y-direction. In this manner, the portion of the skin 13' present in front of the beam exit window 15' is scanned according to a predetermined line pattern.

During scanning the auxiliary laser source 59 generates the auxiliary laser beam 61, which is a continuously pulsed laser beam with a frequency which is sufficiently high for the scanning purpose. When during scanning the auxiliary laser beam 61 hits a hair 3' present on the skin 13', the hair 3' will irradiate a characteristic two-photon fluorescence signal under the influence of the impacting auxiliary laser beam 61. The principle of two-photon fluorescence is generally known, e.g. from the technical field of microscopy, and will not be further explained in detail here. The person skilled in the art will also be able to determine the wavelength, the energy density, and the focus diameter of the auxiliary laser source 59 necessary to achieve the desired two-photon fluorescence signals from the hairs 3'. The two-photon fluorescence signal irradiated by the hair 3' has a spectrum which is characteristic for hair tissue. The signal is detected by the detector 65, which supplies an output signal u_{D} to the control unit 43'. The moment the control unit 43' detects the presence of a hair 3' on the basis of the output signal u_{D}, the control unit 43' activates the laser source 9' via its third output signal u_{LS}, so that the laser source 9' generates a laser pulse in the target position 22' coinciding with the target position 63 of the auxiliary laser beam 61, and so that the hair 3' is cut in said target position 22'. The control unit 43' of the device 1' is considerably simpler and faster than the control unit 43 of the device 1, because the control unit 43' does not have to analyse and interpret a rather complex image of the skin portion present before the beam exit window 15' as the control unit 43 of the device 1 should do.

It is noted that, instead of being designed to detect a characteristic two-photon fluorescence signal, the auxiliary laser source 59 and the detector 65 of the optical detecting system 31' may alternatively be designed to detect another kind of characteristic optical signal coming from a hair under the influence of the impacting auxiliary laser beam. An example is an optical signal which is characteristic for the optical absorption properties of the hair tissue.

It is finally noted that the invention also covers other types of devices for the treatment of skin by means of a radiation beam. Examples of such devices are devices for the medical or cosmetic treatment of birthmarks, such as naevus vinosus and naevus pigmentosus, present on the skin, psoriasis, or aberrations of blood vessels present in the skin, such as varicose veins.

## Claims

1. A device (1) for the treatment of skin by means of a radiation beam, which device comprises a radiation source (9) for generating the radiation beam, a beam exit window (15), an optical detecting system (31) for determining a target position of the radiation beam relative to the skin, and a beam manipulator (17) for positioning the radiation beam in the target position, the radiation beam and the detecting system having a first (45) and a second (47) optical axis, respectively, **characterized in that**, seen from the radiation source towards the beam exit window, the first and the second optical axis coincide starting at a position between the radiation source and the beam manipulator.

2. A device as claimed in claim 1, **characterized in that** the device comprises a dichroic beam splitter in said position between the radiation source and the beam manipulator.

3. A device as claimed in claim 1, **characterized in that** the optical detecting system comprises an image sensor, the second optical axis extending from the image sensor towards the beam exit window.

4. A device as claimed in claim 1, **characterized in that** the optical detecting system comprises an auxiliary radiation source for generating an auxiliary radiation beam, and a detector for detecting a characteristic optical signal coming from a hair under the influence of the auxiliary radiation beam, the second optical axis extending from the auxiliary radiation source towards the beam exit window.

5. A device as claimed in claim 4, **characterized in that** the detector is a two-photon fluorescence detector for detecting a characteristic two-photon fluorescence signal irradiated by a hair under the influence of the auxiliary radiation beam.

6. A device as claimed in claim 1, 2, 3, 4 or 5, **characterized in that** the device is a device for shortening hairs, wherein the radiation source is a laser source.

## Patentansprüche

1. Vorrichtung (1) zur Hautbehandlung mittels eines Strahlungsbündels, wobei diese Vorrichtung zum erzeugen des Strahlungsbündels eine Strahlungsquelle (9), ein Strahlungsausgangsfenster (15), ein optisches Detektionssystem (31) zum Ermitteln einer Zielstelle des Strahlungsbündels in Bezug auf die Haut und ein Bündelstellglied (17) zum Positionieren des Strahlungsbündels auf die Zielstelle aufweist, wobei das Strahlungsbündel und das Detektionssystem eine erste (41) bzw. eine zweite optische Achse (47) haben, **dadurch gekennzeichnet, dass**, von der Strahlungsquelle in Richtung des Bündelaustrittfensters gesehen, die erste und die zweite optische Achse, ausgehend von einer Stelle zwischen der Strahlungsquelle und dem Bündelstellglied, zusammenfallen.

2. Vorrichtung nach Anspruch 1, **dadurch gekennzeichnet, dass** die Vorrichtung einen Kaltlichtbündelspalter an der genannten Stelle zwischen der Strahlungsquelle und dem Bündelstellglied aufweist.

3. Vorrichtung nach Anspruch 1, **dadurch gekennzeichnet, dass** das optische Detektionssystem einen Bildsensor aufweist, wobei die zweite optische Achse sich von dem Bildsensor in Richtung des Bündelaustrittfensters erstreckt.

4. Vorrichtung nach Anspruch 1, **dadurch gekennzeichnet, dass** das optische Detektionssystem zum Erzeugen eines Hilfsstrahlungsbündels eine Hilfsstrahlungsquelle aufweist, sowie einen Detektor zum Detektieren eines charakteristischen optischen Signals herrührend von einem Haar, und zwar unter dem Einfluss des Hilfsstrahlungsbündels, wobei die zweite optische Achse sich von der Hilfsstrahlungsquelle in Richtung des Bündelaustrittsfensters erstreckt.

5. Vorrichtung nach Anspruch 4, **dadurch gekennzeichnet, dass** der Detektor ein Doppelphoton-Fluoreszenzdetektor zum Detektieren eines von einem Haar unter dem Einfluss des Hilfsstrahlungsbündels ausgestrahlten charakteristischen Doppelphoton-Fluoreszenzsignals.

6. Vorrichtung nach Anspruch 1, 2, 3, 4 oder 5, **dadurch gekennzeichnet, dass** die Vorrichtung eine Vorrichtung zum Kürzen von Haaren ist, wobei die Strahlungsquelle eine Laserquelle ist.

## Revendications

1. Dispositif (1) pour le traitement de la peau au moyen d'un faisceau de rayonnement, lequel dispositif comprend une source de rayonnement (9) pour générer le faisceau de rayonnement, une fenêtre de sortie de faisceau (15), un système de détection optique (31) pour déterminer une position cible du faisceau de rayonnement par rapport à la peau et un manipulateur de faisceau (17) pour positionner le faisceau de rayonnement dans la position cible, le faisceau de rayonnement et le système de détection ayant un premier (45) et un deuxième (47) axe optique, respectivement, **caractérisé en ce que**, vu à partir de la source de rayonnement vers la fenêtre de sortie de faisceau, le premier et le deuxième axe optique coïncident à partir d'une position entre la source de rayonnement et le manipulateur de faisceau.

2. Dispositif selon la revendication 1, **caractérisé en ce que** le dispositif comprend un diviseur de faisceau dichroïque dans ladite position entre la source de rayonnement et le manipulateur de faisceau.

3. Dispositif selon la revendication 1, **caractérisé en ce que** le système de détection optique comprend un capteur d'image, le deuxième axe optique s'étendant à partir du capteur d'image vers la fenêtre de sortie de faisceau.

4. Dispositif selon la revendication 1, **caractérisé en ce que** le système de détection optique comprend une source de rayonnement auxiliaire pour générer un faisceau de rayonnement auxiliaire et un détecteur pour détecter un signal optique caractéristique en provenance d'un poil sous l'influence du faisceau de rayonnement auxiliaire, le deuxième axe optique s'étendant à partir de la source de rayonnement auxiliaire vers la fenêtre de sortie de faisceau.

5. Dispositif selon la revendication 4, **caractérisé en ce que** le détecteur est un détecteur de fluorescence à deux photons pour détecter un signal de fluorescence caractéristique à deux photons qui est irradié par un poil sous l'influence du faisceau de rayonnement auxiliaire.

6. Dispositif selon les revendications 1, 2, 3, 4 ou 5, **caractérisé en ce que** le dispositif est un dispositif pour raccourcit des poils dans lequel la source de rayonnement est une source laser.
